# EUROPÄISCHE PATENTSCHRIFT

(11) **EP 2 523 094 B1**
(45) Veröffentlichungstag und Bekanntmachung des Hinweises auf die Patenterteilung: **08.05.2019**
(21) Anmeldenummer: 12175890.8
(22) Anmeldetag: 29.07.2004
(51) Int. Cl.: G06F 3/14, G06F 1/16

(54) **Anzeige- und Steuervorrichtung für medizintechnische Geräte**
Display and control device for medical equipment
Dispositif d'affichage et de commande pour appareils médico-chirurgicaux

(30) Priorität: 29.07.2003 DE 10334516
(43) Veröffentlichungstag der Anmeldung: 14.11.2012
(62) Teilanmeldung aus: 04763602.2
(73) Patentinhaber: Sorin Group Deutschland GmbH, 80939 München (DE)
(72) Erfinder: Knott, Erwin, 85586 Poing (DE); Penka, Ottmar, 81245 München (DE); Hahn, Andreas, 82335 Berg (DE)
(74) Vertreter: Hoffmann Eitle

(56) Entgegenhaltungen:
- US-A- 5 752 931
- US-A1- 2003 067 437
- US-B2- 6 496 359
- Anonymous: "SIII System", Stöckert Instrumente GmbH, München , 1 August 2001 (2001-08-01), XP055082779, Retrieved from the Internet: URL:http://www.perfusion.com/cgi-bin/absol utenm/articlefiles/stockertSIII/StockertSI II_Manual.pdf [retrieved on 2013-10-07]

## Beschreibung

Die vorliegende Erfindung betrifft eine Anzeige- und Steuervorrichtung für medizintechnische Geräte, insbesondere Lebenserhaltungssysteme wie Herz-Lungen-Maschinen oder Beatmungsgeräte.

Um medizintechnische Geräte sicher bedienen und überwachen zu können, müssen an diesen Geräten Anzeige- und Bedienvorrichtungen vorgesehen werden, die es dem Benutzer gestatten, in übersichtlicher Form den Betriebszustand des Geräts zu erfassen und mit Hilfe von Bedienelementen in dem Betrieb des Geräts einzugreifen. So sind zum Beispiel bei Herz-Lungen-Maschinen (im Folgenden auch: HLM) Anzeige- und Bedienelemente für die verschiedenen Einheiten und Komponenten, z.B. die Blutpumpen oder den Oxygenator vorgesehen, über die der Benutzer den Betrieb der Einheiten der HLM überwachen und beeinflussen kann. Daneben sind Anzeige- und Bedienelemente für verschiedene Sensoren, beispielsweise Füllstandssensoren, Temperatursensoren oder Luftblasendetektoren vorgesehen, an denen der Benutzer einen Sensormesswert ablesen und Grenzwerte einstellen kann, bei deren Über- bzw. Unterschreiten ein Alarm oder eine andere Aktion ausgelöst wird. Bei HLM werden diese Anzeige- und Bedienelemente neuerdings oft in Form eines Anzeige- und Bedienpaneels zusammengefasst, so dass der Benutzer an einem Ort in übersichtlicher Weise den Betrieb der gesamten Herz-Lungen-Maschine überwachen und steuern kann.

Das S3-System der Stöckert Instrumente GmbH München, zum Beispiel beschrieben in den Operating Instructions, zeigt eine Anzeige- und Steuervorrichtung, zum Beispiel für ein modulares Perfusionssystem, bei dem ein Anzeige- und Bedienpanel vorgesehen ist, an das sechs oder zehn vorab konfigurierte Module installiert werden können.

Anzeige- und Bedienpaneele der hier angesprochenen Art werden in zunehmendem Masse unter Verwendung programmgesteuerter Bildschirme und Tastatureinheiten realisiert. In jüngster Zeit werden diese Benutzerschnittstellen (GUI = Graphical User Interface) in Form von LCD-Anzeigegeräten in Kombination mit einer berührungsempfindlichen Abdeckung der LCD-Anzeigeoberfläche verwendet (Touch Screen). Das bedeutet, der Benutzer kann nicht nur Werte auf der Anzeige ablesen, sondern auch auf der Anzeige bildhaft dargestellte Taster, Schalter und Regler betätigten, indem er die Anzeigenoberfläche im Bereich der Darstellung eines Tasters, Schalters oder Reglers berührt.

Der Einsatz derartiger Touch-Screen-GUI-Einheiten ermöglicht eine einfache Bedienung auch komplexer Systeme, insbesondere durch Reduktion der Bedienelemente und dadurch gesteigerte Übersichtlichkeit, durch kontext-abhängige Gestaltung der Bedienmöglichkeiten und durch situationsabhängige Hilfestellungen durch das System. Auch fertigungsseitig bieten die GUI-Einheiten dieser Art Vorteile, insbesondere bei höheren Stückzahlen. Jedoch ist nachteilig, dass bei Verwendung einer GUI-Einheit ein Anzeigendefekt zu einem vollständigen Verlust der Systemkontrolle führen kann. Eine Interaktion über die GUI-Einheit mit dem Benutzer ist bei einem Totalausfall nicht mehr möglich. Auch Teilausfälle können zu einer nicht akzeptablen Beeinträchtigung der Bedienbarkeit des medizintechnischen Geräts führen. Dabei ist zu beachten, dass berührempfindliche Oberflächen systembedingt anfällig für mechanische Defekte sind. Bei komplexen Anwendungen ist überdies ein aufwendiger Entwicklungsprozess für die GUI-Einheit und deren Ansteuerung erforderlich, insbesondere bei der Realisierung sprachspezifischer Varianten.

Dies hat zur Folge, dass derartige Anzeige- und Bedienvorrichtungen für sicherheitskritische Anwendungen in Lebenserhaltungssystemen, wie Herz-Lungen-Maschinen oder Beatmungsgeräten nur mit erheblichem Aufwand (redundante Bedienfelder, speziell angepasste Betriebssysteme etc.) eingesetzt werden können. Wo eine redundante Auslegung nicht möglich oder sinnvoll ist, muss die Anzeige- und Bedienvorrichtung als Austauschteil bereitgestellt werden, das bei Versagen der eingesetzten Anzeige- und Bedienvorrichtung gegen diese ausgetauscht werden kann. Im Hinblick auf die Kosten für die Bereitstellung eines Austauschpaneels ist diese Vorgehensweise doch unbefriedigend.

US 6 496 359 B2 beschreibt einen Computer, der aus individuellen kachelartig angeordneten Platten gebildet wird. Jede Platte umfasst eine Anzeigenoberfläche, eine Prozessor, einen Speicher und Kommunikationsmittel zur Bereitstellung von Kommunikationen mit benachbarten Platten oder der Trägerstruktur, auf der die Platten angeordnet sind.

Vor diesem Hintergrund hat die Erfindung zum Ziel eine Anzeige- und Bedienvorrichtung für medizintechnische Geräte, insbesondere Herz-Lungen-Maschinen oder andere Lebenserhaltungssysteme wie etwa Beatmungssysteme anzugeben, bei der im Falle eines Defekts die Fortsetzung des Betriebs auf wirtschaftliche Weise gewährleistet werden kann.

Dieses Ziel wird erreicht durch eine Anzeige- und Bedienvorrichtung mit den Merkmalen des Patentanspruchs 1. Vorteilhafte Ausgestaltungen ergeben sich aus den Unteransprüchen.

Erfindungsgemäß werden mehrere Touch-Screen-GUI-Einheiten zu einer modularen Anzeige- und Bedienvorrichtung zusammengesetzt. Die GUI-Einheiten sind in ihrem Aufbau praktisch identisch und als Steckmodul ausgeführt, das an verschiedenen Positionen eines Basispaneels angebracht werden kann. Die Festlegung der Anzeige- und Bedienfunktion der einzelnen Touch-Screen-GUI-Einheit erfolgt durch eine Konfigurationseinrichtung, mit der die Touch-Screen-GUI-Einheit über ein Bussystem (in der Basis) verbunden ist und von der sie Konfigurationsdaten erhält. Die Konfigurationsdaten werden bei Anschliessen einer Touch-Screen-GUI-Einheit an das Bussystem an die Touch-Screen-GUI-Einheit übermittelt und entsprechen der Ansteckposition auf der Basiseinheit. Die Konfigurationsdaten bestimmen über die Anzeigeninhalte und Eingabebereiche die Anzeige- und Bedienfunktion der Touch-Screen-GUI-Einheit.

Im Folgenden wird die Erfindung anhand eines Ausführungsbeispiels unter Bezugnahme auf die Zeichnungen genauer erläutert, in denen zeigt:
- Fig. 1:: schematisch den Aufbau einer HLM als ein für die Erläuterung der Erfindung besonders geeignetes Beispiel eines Lebenserhaltungssystems im Speziellen und eines medizintechnischen Geräts im Allgemeinen;
- Fig. 2:: eine perspektivische Darstellung des Aufbaus einer erfindungsgemäßen Anzeige- und Bedienvorrichtung;
- Fig. 3:: eine perspektivische Darstellung der Vorderseite einer Anzeige/Bedieneinheit gemäß der Erfindung;
- Fig. 4:: eine perspektivische Darstellung der Rückseite einer Anzeige/Bedieneinheit gemäß der Erfindung;
- Fig. 5:: eine perspektivische Darstellung des Aufbaus einer Basiseinheit gemäß der Erfindung;
- Fig. 6:: eine Darstellung einer Basiseinheit und einer daran angeordneten Anzeige/Bedieneinheit gemäß der Erfindung;
- Fig. 7A bis 7E:: Beispiele für auf Konfigurationsdaten zurückgehende Anzeigeninhalte einer Anzeige/Bedieneinheit gemäß der Erfindung.

Die Erfindung wird durch die angehängten Ansprüche definiert. Jeder Verweis in der folgenden Beschreibung auf Ausführungsformen, Gegenstände, Aspekte und/oder Beispiele, die nicht in den angehängten Ansprüchen enthalten sind, werden nicht als Teil der vorliegenden Erfindung angesehen.

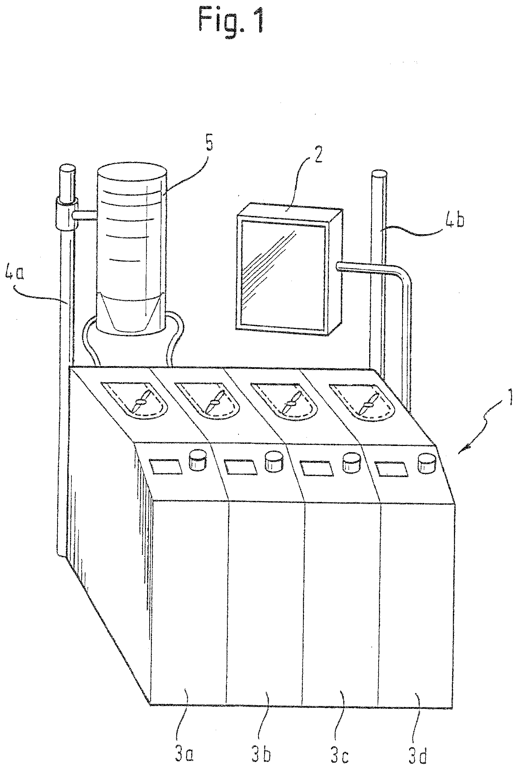

In Fig. 1 ist schematisch eine Herz-Lungen-Maschine 1 dargestellt, an der exemplarisch eine Anzeige- und Bedienvorrichtung 2 erläutert werden soll, wie sie bei medizintechnischen Geräten, insbesondere Lebenserhaltungssystemen wie Herz-Lungen-Maschinen und Beatmungsgeräten anzutreffen ist.

Die in Fig. 1 gezeigte Herz-Lungen-Maschine 1 umfasst vier Blutpumpen 3a, 3b, 3c und 3d, die nebeneinander angeordnet sind. Die Gehäuse der Blutpumpen bilden den Grundkörper der HLM 1. An Befestigungsmasten 4a und 4b können weitere Aggregate oder Einheiten der HLM 1 angeordnet sein, wie beispielsweise das schematisch angedeutete Blutreservoir 5. Überdies sind bei einer HLM üblicherweise Sensoren (nicht dargestellt) vorhanden, um verschiedene Messwerte wie Temperaturen, Drücke und Flüsse zu erfassen. Ferner sind üblicherweise Blasendetektoren (nicht dargestellt) vorgesehen, über die sichergestellt wird, dass dem Patienten kein Luftblasen enthaltendes Blut zugeführt wird. Es ist nicht beabsichtigt, die Einheiten oder Komponenten einer HLM hier vollständig aufzuzählen. Die zuvor erwähnten Einheiten sind lediglich exemplarisch anhand einer HLM, aber auch für die übrigen Lebenserhaltungssysteme bzw. medizintechnischen Geräte erwähnt. Jedoch eignet sich eine HLM ebenso wie Beatmungsgeräte in besonderem Maße für die Anwendung der Erfindung.

An der in Fig. 1 gezeigten HLM 1 ist die bereits angesprochene Anzeige- und Bedienvorrichtung 2 vorgesehen, mit der die Einheiten oder Komponenten der HLM 1 überwacht und gesteuert werden können. Dazu zeigt die Anzeige- und Bedienvorrichtung 2 verschiedene Werte an, teilweise Messwerte der Sensoren und Detektoren, teilweise Betriebsparameter der Einheiten, wie beispielsweise die Drehzahl der Blutpumpen oder auch andere Werte, beispielsweise Zeiten. Der Benutzer erhält über die Anzeige der Werte einen Überblick über den Betriebszustand der HLM 1 und kann über die Anzeige- und Bedienvorrichtung 2 steuernd eingreifen, indem er die in der Anzeige- und Bedienvorrichtung bildhaft angebotenen Bedienelemente benutzt.

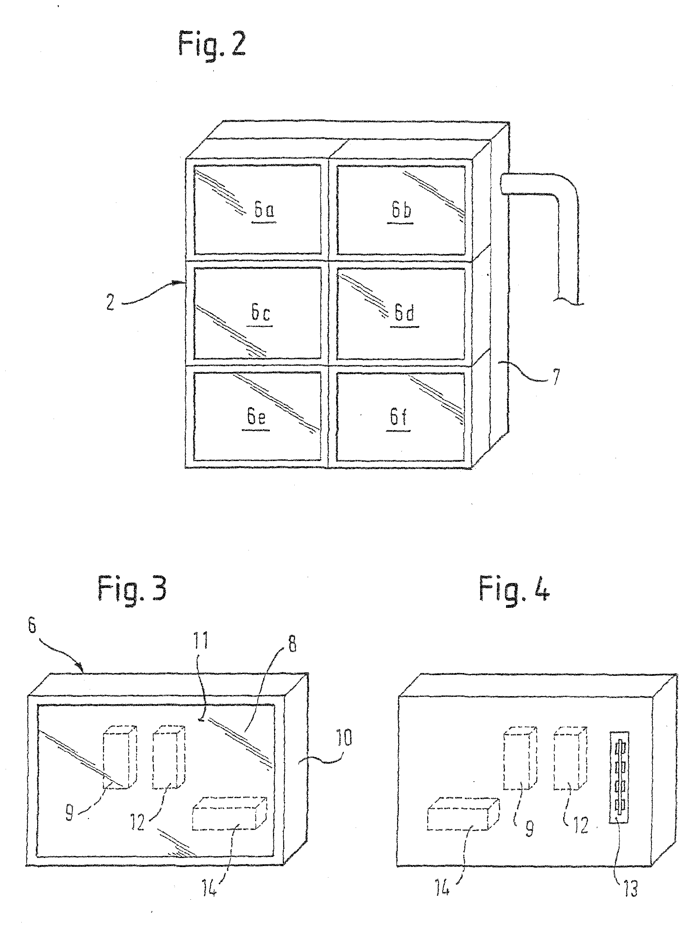

In Figur 2 ist eine erfindungsgemäße Anzeige- und Bedienvorrichtung 2 dargestellt; sie umfasst mehrere Anzeige-/Bedieneinheiten 6a bis 6f, die auf einer Basiseinheit 7 angeordnet sind. Wie Figur 3 zeigt, in der eine Vorderansicht einer erfindungsgemäßen Anzeige-/Bedieneinheit 6 dargestellt ist, umfasst jede Anzeige-/Bedieneinheit 6 eine flache Anzeigeeinrichtung 8, die eine Vielzahl von ansteuerbaren Bildpunkten aufweist und auf der sowohl Messwerte in Form von Zahlenwerten als auch Text oder grafische Bestandteile angezeigt werden können. Dazu ist die Anzeige-/Bedieneinheit 6 mit einer Anzeigesteuereinrichtung 9 ausgestattet, die die Bildpunkte der Anzeigeeinrichtung 8 auf der Grundlage von zugeführten Daten ansteuert. Die Anzeigesteuereinrichtung 9 ist vorzugsweise in einem Gehäuse 10 der Anzeige-/Bedieneinheit 6 vorgesehen, weshalb sie in der Darstellung der Figur 3 gestrichelt wiedergegeben ist. Um eine Eingabemöglichkeit zu schaffen weist jede Anzeige-/Bedieneinheit eine transparente Eingabeeinrichtung 11 auf, die auf der einem Betrachter zugewandten Oberfläche der Anzeigeeinrichtung 8 angeordnet ist und die mit einer Eingabeauswerteeinrichtung 12 in Verbindung steht, die die über die Eingabeeinrichtung 11 erfolgenden Eingaben auswertet. Auch die Eingabeauswerteeinrichtung 12 ist vorzugsweise in dem Gehäuse 10 untergebracht und deshalb gestrichelt dargestellt. Insgesamt wird auf diese Weise eine Touch-Screen-Anzeige/Eingabeeinheit 6 geschaffen, die einerseits Informationen anzeigen (8, 9) und andererseits Eingaben entgegennehmen (11, 12) kann. Weitere Anzeige- oder Bedienelemente sind erfindungsgemäß nicht erforderlich, können aber vorgesehen werden. Insbesondere kann ein Ein/AusSchalter vorgesehen werden, damit beim Aufstecken der Anzeige/Bedieneinheit 6 auf die Basiseinheit 7, die Anzeige/Bedieneinheit 6 nicht aktiv ist.

Damit die Daten für die Anzeigeninhalte zur Anzeigesteuereinrichtung 9 übertragen und die Eingaben von der Eingabeauswerteeinrichtung 12 weitergeleitet werden können, umfasst die Anzeige/Bedieneinheit 6 eine Anschlusseinrichtung 13, die in Figur 4 - einer Rückansicht einer Anzeige/Bedieneinheit - gezeigt ist. Mit der Anschlusseinrichtung 13 sind die Anzeigesteuereinrichtung 9 und die Eingabeauswerteeinrichtung 12 verbunden, so dass die erfindungsgemäße Anzeige/Bedieneinheit 6 an einen elektrischen Bus anschließbar ist.

Bei dem hier angesprochenen elektrischen Bus handelt es sich um ein Verbindungssystem, das es an den Bus angeschlossenen elektrischen Geräten erlaubt, mit anderen an den Bus angeschlossenen Geräten zu kommunizieren. Das bedeutet im Wesentlichen, dass Werte oder andere Informationen gezielt von einem Busgerät zu einem anderen Busgerät übertragen, d.h. gesendet und empfangen werden können.

In Abhängigkeit von der Art des eingesetzten Busses kann zweckdienlicherweise eine Buskommunikationseinrichtung 14 vorgesehen werden, die einerseits die Verbindung zum Bus gewährleistet und andererseits mit der Anzeigesteuereinrichtung 9 und der Eingabeauswerteeinrichtung 12 verbunden ist, wodurch die Anzeigesteuereinrichtung 9 und die Eingabeauswerteeinrichtung 12 nicht für einen direkten Anschluss an den Bus ausgelegt werden müssen.

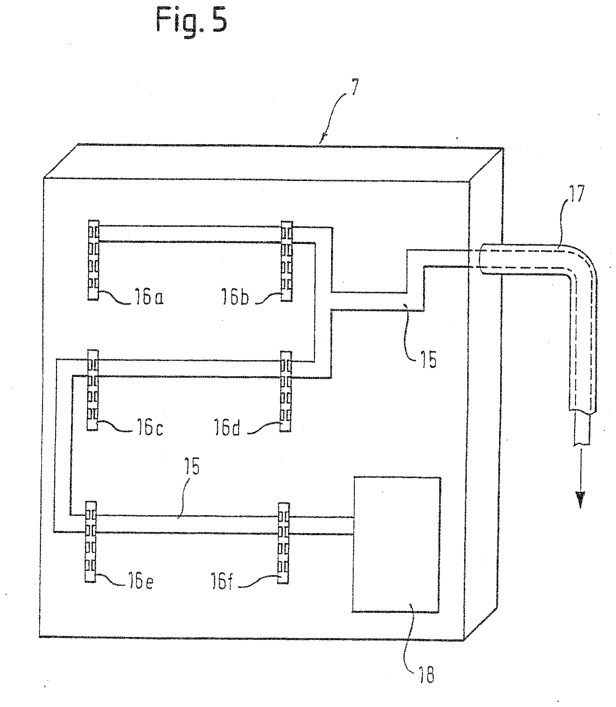

Wie Figur 5 zeigt, ist der Bus 15 in der Basiseinheit 7 der erfindungsgemäßen Anzeige- und Bedienvorrichtung 2 vorgesehen. Er dient, wie bereits ausgeführt, der Kommunikation von an den Bus angeschlossenen Einheiten. Damit die Anzeige/Bedieneinheiten 6a bis 6f an den Bus angeschlossen werden können, sind an der Basiseinheit 7 geeignete Verbindungseinrichtungen 16a bis 16f vorgesehen. Jede Verbindungseinrichtung 16 in der Basiseinheit 7 ist an die Anschlusseinrichtung 13 der Anzeige-/Bedieneinheiten 6 angepasst, so dass durch Zusammenführen einer Anschlusseinrichtung 13 und einer Verbindungseinrichtung 16, vorzugsweise nach dem Stecker-Buchse-Prinzip, eine Anzeige/Bedieneinheit 6 an den Bus 15 anschließbar ist, wenn die Anzeige/Bedieneinheit 6 an der Basiseinheit 7 angeordnet wird.

Die Verbindung zwischen Verbindungseinrichtung 16 und Anschlusseinrichtung 13 ist entweder derart, dass auch eine ausreichende mechanische Halterung der Anzeige/Bedieneinheit 6 an der Basiseinheit 7 erreicht wird, oder derart, dass zusätzliche Vorkehrungen zur mechanische Halterung getroffen werden müssen, die hier aber nicht näher erläutert werden. Im Grunde eignet sich jede von einem Benutzer einfach herstellbare und wieder lösbare Halterung der Anzeige/Bedieneinheit 6 an der Basiseinheit 7, wobei die Anforderungen zu beachten sind, die im Umfeld der medizintechnischen Geräte und insbesondere der Lebenserhaltungssysteme existieren.

Der Bus 15 ist aus der Basiseinheit 7 herausgeführt, beispielsweise, wie in Figur 5 gezeigt, durch die Stütze 17 der Anzeige- und Bedienvorrichtung 2. Durch die Stütze 17 führt der Bus in das medizintechnische Gerät, wie beispielsweise bei der HLM in Figur 1 erkennbar, so dass die Einheiten und Aggregate des medizintechnischen Geräts auch an den Bus angeschlossen werden können.

In der Basiseinheit 7 ist ferner eine Konfigurationseinheit 18 vorgesehen, die auch mit dem elektrischen Bus 15 verbunden ist. Die Konfigurationseinheit 18 übernimmt unmittelbar nach dem Anschließen einer Anzeige/Bedieneinheit 6 an den Bus, also in der Regel beim Anstecken der Anzeige/Bedieneinheit 6 an die Basiseinheit 7, die Aufgabe, der Anzeige/Bedieneinheit 6 erste Daten zu übermitteln. Dabei handelt es sich um Konfigurationsdaten, die der Anzeige/Bedieneinheit 6 mitteilen, welche Aufgabe sie in der Gesamtheit der Anzeige- und Bedienvorrichtung 2 zu übernehmen hat. Die Konfiguration der Anzeige/Bedieneinheit 6 ist ein wichtiger Aspekt der erfindungsgemäßen Anzeige- und Bedienvorrichtung 2, da dadurch eine Anzeige/Bedieneinheit 6 unterschiedlich verwendet werden kann, da erst die Konfigurationsdaten festlegen, wofür die Anzeige/Bedieneinheit 6 eingesetzt wird. Im Wesentlichen handelt es sich bei den über den elektrischen Bus 15 übermittelten Konfigurationsdaten um Informationen (Anweisungen), die die Anzeigeninhalte und die Eingabebereiche der Anzeige-/Bedieneinheit 6 festlegen. Die Anzeigesteuereinrichtung 9 der Anzeige/Bedieneinheit 6 muss die durch die Konfigurationsdaten übermittelten Anzeigeinhalte in der Anzeige 8 der Anzeige/Bedieneinheit 6 darstellen (Figur 3); die Eingabeauswerteeinrichtung 12 muss dementsprechend die durch die Konfigurationsdaten als Eingabebereiche definierten Abschnitte auf der Oberfläche 11 der Anzeige 8 auswerten und auf Eingaben durch den Benutzer überwachen.

Da die Anzeige/Bedieneinheiten 6 einer erfindungsgemäßen Anzeige- und Bedienvorrichtung 2 praktisch identisch aufgebaut sind, kann jede Anzeige/Bedieneinheit 6 an jeder Stelle (16a bis 16f) der Basiseinheit 7 eingesetzt werden. Das bedeutet, dass während der Benutzung des medizintechnischen Geräts, beispielsweise der HLM im Operationssaal in der Regel nur eine Anzeige/Bedieneinheit als Austauschteil vorgehalten werden muss, da bei Ausfall irgendeiner Anzeige/Bedieneinheit in der Anzeige- und Bedienvorrichtung die redundante Anzeige/Bedieneinheit als Ersatz eingesetzt werden kann. Darüber hinaus kann eine bereits verwendete Anzeige/Bedieneinheit außer Betrieb genommen werden, wenn ihre Funktion von untergeordneter Bedeutung ist (z.B. Zeitanzeige) und an die Stelle einer ausgefallenen Anzeige/Bedieneinheit gesetzt werden, die kritische Überwachungs- Steuerungsfunktionen unterstützt, die für den Benutzer unverzichtbar sind.

Neben der Möglichkeit eine Anzeige/Bedieneinheit als kostengünstiges Austauschteil bereitzuhalten, schafft die Erfindung die Möglichkeit, durch redundante Auslegung der Konfigurationseinrichtung 18 einen sicheren Betrieb zu gewährleisten. Auch die redundante Auslegung der Konfigurationseinrichtung 18 stellt einen akzeptablen Aufwand dar, wodurch die Erfindung ermöglicht wird. Daneben ist zu beachten, dass während des Betriebs der Anzeige- und Bedienvorrichtung gemäß der Erfindung die Konfigurationseinrichtung 18 keine weiteren Funktionen übernimmt. Das bedeutet, dass ihr Ausfall während des Betriebs nach abgeschlossener Konfiguration der Anzeige/Bedieneinheiten zunächst keine Beeinträchtigung der Gesamtfunktion der Anzeige- und Bedienvorrichtung gemäß der Erfindung darstellt. Dennoch sollte eine Störung der Konfigurationseinrichtung 18 dem Benutzer signalisiert werden, da bei gestörter Konfigurationseinrichtung 18 ein Austausch einer Anzeige/Bedieneinheit 6 nicht mehr möglich ist.

Im Folgenden wird beispielhaft beschrieben, in welcher Form Konfigurationsdaten von der Konfigurationseinrichtung 18 an eine Anzeige/Bedieneinheit 6 übertragen werden und in welcher Art und Weise die Konfigurationsdaten in der Anzeige/Bedieneinheit 6 verarbeitet werden.

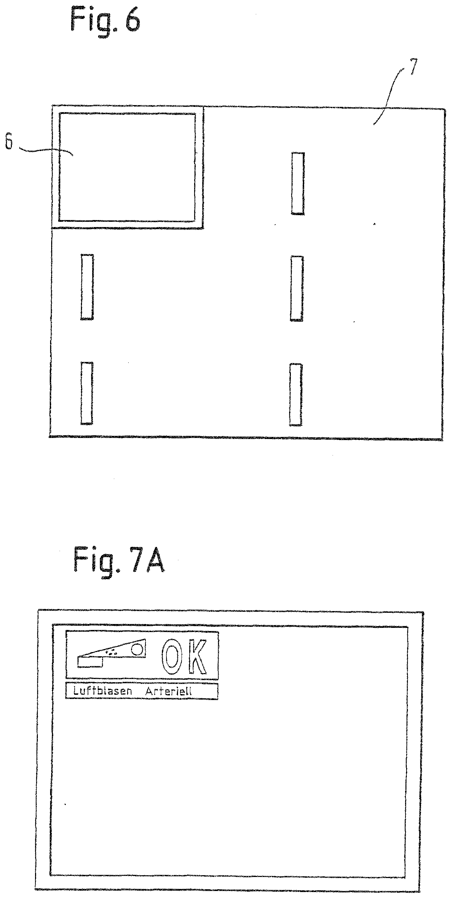

Dazu zeigt Figur 6 eine Basiseinheit 7, an der eine Anzeige/Bedieneinheit 6 angesteckt ist. Wenn eine Anzeige/Bedieneinheit 6 an der Basiseinheit 7 angeordnet und durch Zusammenführen der Anschlusseinrichtung 13 und der Verbindungseinrichtung 16 an den elektrischen Bus 15 in der Basiseinheit 7 angeschlossen wird, wird der Konfigurationseinrichtung 18 über den Bus 15 signalisiert, dass und an welcher Verbindungseinrichtung 16a bis 16f eine Anzeige/Bedieneinheit 6 angeschlossen wurde. Da die Position der Verbindungseinrichtung 16a bei dem hier beschriebenen Ausführungsbeispiel vorgibt, welche Anzeigeninhalte dargestellt und welche Eingaben entgegengenommen werden, übermittelt die Konfigurationseinrichtung 18 über den Bus 15 solche Konfigurationsdaten an die Anzeige/Bedieneinheit 6, die der Ansteckposition auf der Basiseinheit 7 der Anzeige- und Bedienvorrichtung 2 entsprechen.

Einzelheiten des Konfigurationsvorgangs werden im Folgenden anhand der Figuren 7A bis E erläutert. Dabei wird der Einsatz einer erfindungsgemäßen Anzeige- und Bedienvorrichtung 2 an einer Herz-Lungen-Maschine (vgl. Figur 1) betrachtet, die neben anderen Einheiten zum Beispiel einen Sensor für die Füllstandsüberwachung einem Blutreservoirs und einen Detektor für Luftblasen in der arteriellen Zuleitung, sowie Temperatursensoren für das in das Blutreservoir eintretende und das aus dem Blutreservoir austretende Blut besitzt. Diese Sensoren und Detektoren, aber auch die anderen Einheiten und Komponenten der HLM sind an den Bus 15 angeschlossen. Die Messwerte dieser Sensoren/Detektoren sollen mit Hilfe einer erfindungsgemäßen Anzeige/-Bedienvorrichtung 2 visualisiert werden, wobei beispielhaft festgelegt ist, dass die Darstellung immer im Bereich der Verbindungseinrichtung 16a auf der Basiseinheit 7 der Anzeige- und Bedienvorrichtung 2 erfolgt.

Wenn nun eine Anzeige/Bedieneinheit 6 an der Position 16a der Basiseinheit 7 angeordnet und eine Verbindung zum Bussystem 15 hergestellt wird, übermittelt die Konfigurationseinrichtung 18 der Anzeige/Bedieneinheit 6 Konfigurationsdaten, die die Anzeigeinhalte und die Eingabebereiche festlegen.

In einem ersten Schritt übermittelt die Konfigurationseinrichtung der Anzeige/Bedieneinheit beispielsweise die Konfigurationsdaten:
*(1) DispletPos*=*"1*"
(2) *Title Color*=*"Blau" "Luftblasen Arteriell"*
   und veranlasst die Anzeige/Bedieneinheit 6 in der obersten von drei vordefinierten Zeilen in der linken Hälfte des Anzeigebereichs 8 eine blaue Titelzeile mit dem Text "Luftblasen arteriell" anzuzeigen, was in Figur 7A gezeigt ist. Bei dem Anzeigebereich handelt es sich um eine logische Unterteilung der ansonsten vorteilhafterweise kontinuierlichen und nicht unterteilten Anzeige 8 der Anzeige/Bedieneinheit 6. In einem zweiten Schritt übermittelt die Konfigurationseinrichtung 18 der Anzeige/Bedieneinheit 6 die Konfigurationsdaten
*(3) Displconl Pic="BubbleDetector"*
   und veranlasst die Anzeige/Bedieneinheit 6 oberhalb der Titelzeile "Luftblasen arteriell" ein schematisches Bild für einen Luftblasendetektor anzuzeigen, was auch aus Figur 7A entnehmbar ist. In einem dritten Schritt übermittelt die Konfigurationseinrichtung 18 der Anzeige/Bedieneinheit 6 die Konfigurationsdaten
*(4) ValueCan="0000062"*
   die die Anzeige/Bedieneinheit 6 veranlassen, Werte des Busgeräts mit der Identifikation 0000062 über den elektrischen Bus 15 entgegenzunehmen. Diese Identifikation ist beispielsweise dem Luftblasendetektor der HLM zugewiesen worden, so dass die Anzeige/Bedieneinheit 6 durch die übermittelten Konfigurationsdaten dafür konfiguriert wird, Sensorsignale des Luftblasendetektors entgegenzunehmen. In einem vierten Schritt übermittelt die Konfigurationseinrichtung 18 der Anzeige/Bedieneinheit 6 die Konfigurationsdaten
(5) *ValIcon2 Pic="OK" Stat<"10"*
(6) *ValIcon2 Pic*=*"Alarm" Stat>*=*"10"*
   und veranlasst damit die Anzeige/Bedieneinheit 6 die Buchstaben "OK" in dem Bereich über der Titelzeile "Luftblasen arteriell" anzuzeigen, wenn der von dem Luftblasendetektor erhaltene Wert kleiner als 10 ist. Wenn der Wert größer oder gleich 10 ist, enthalten die Konfigurationsdaten das Wort "ALARM", dass die Anzeige/Bedieneinheit 6 anstelle der Buchstaben "OK" anzeigt. In Figur 7A ist die Anzeige "OK" dargestellt.
   Um die Anzeigeinhalte in der rechten Hälfte der obersten Zeile der Anzeige/Bedieneinheit 6 festzulegen, übermittelt die Konfigurationseinrichtung 18 an die Anzeige/Bedieneinheit 6 die folgenden Konfigurationsdaten
(7) *Displet="2"*
(8) *Title Color="Grün" "Reservoir Level"*
(9) *valueCan="000061*"
(10) *ValIcon1 Pic="FullRes" Stat>"75"*
(11) *ValIcon1 Pic="75%Res" Stat>"50"*
(12) *ValIcon1 Pic="50%Res" Stat>"25"*
(13) *ValIcon1 Pic="25%Res" Stat>"5"*
(14) *ValIcon1 Pic="0%Res" Stat>"0"*
(15) *ValIcon2 Pic="OK" Stat>"25"*
(16) *ValIcon2 Pic="LOW" Stat<="25"*

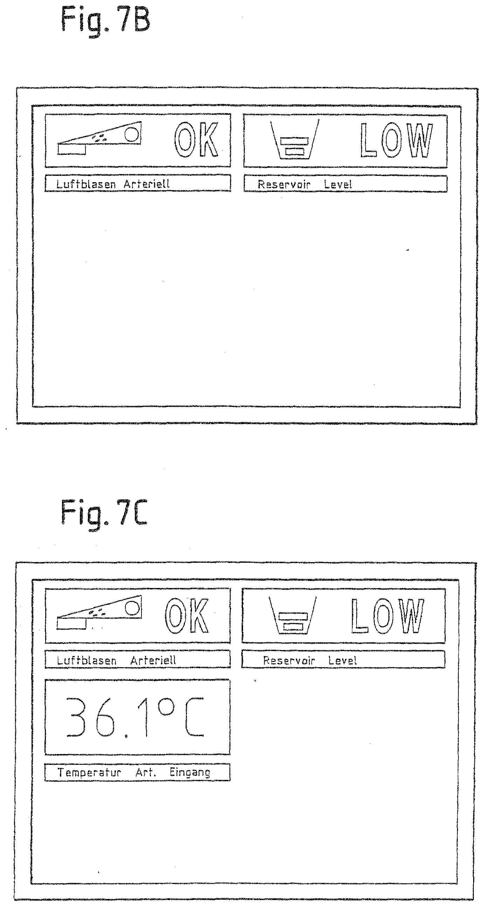

Das Ergebnis dieser Konfigurationsdaten ist in Figur 7B gezeigt, das sich wie folgt ergibt. In den Konfigurationsdaten legt Zeile (7) fest, dass das Anzeigesegment in der rechten Hälfte der obersten Zeile der Anzeige/Bedieneinheit 6 angesprochen wird. Durch die Konfigurationsdaten in Zeile (8) wird veranlasst, dass die Anzeige/Bedieneinheit 6 eine Titelzeile in Grün mit dem Titel "Reservoir Level" anzeigt.

Die Konfigurationsdaten in Zeile (9) veranlassen die Anzeige/Bedieneinheit 6, Daten der an den elektrischen Bus angeschlossenen Einheit mit der Identifikation 000061 entgegenzunehmen. Bei dem hier beschriebenen Ausführungsbeispiel handelt es sich dabei um den Füllstandssensor, der den Füllstand des Blutreservoirs der hier betrachteten HLM als Wert über den elektrischen Bus 15 kommuniziert.

Die Konfigurationsdaten in den Zeilen (10) bis (14) veranlassen die Anzeige/Bedieneinheit 6 in Abhängigkeit von den empfangenen Daten des Füllstandssensors unterschiedliche Grafiken anzuzeigen, die den Füllstand des Reservoirs wiederspiegeln. Dabei werden die unterschiedlichen Grafiken ausgewählt in Abhängigkeit von den Stufen 75%, 50%, 25% und 5%.

Die Konfigurationsdaten in den Zeilen (15) und (16) veranlassen die Anzeige/Bedieneinheit 6 ferner die Buchstaben "OK" im oberen rechten Bereich der Anzeige darzustellen, wenn der Wert des Füllstandssensors größer als 25 ist. Die Buchstaben "OK" werden durch das Wort "LOW" ersetzt, wenn die Werte des Füllstandssensors kleiner oder gleich 25 sind.

Die folgenden Konfigurationsdaten, deren Anzeigeergebnis in Figur 7C gezeigt ist, veranlassen die Anzeige/Bedieneinheit 6 in der linken Hälfte der zweiten Zeile de Anzeige 8 eine Titelzeile in gelber Farbe mit der Bezeichnung "Temperatur Art. Eingang" darzustellen und darüber einen Temperaturwert mit drei Stellen und Dezimalpunkt und die Einheit "°C", wobei die darzustellenden Werte von einem Temperatursensor mit der Identifikation 000060 stammen.
(17) *Displet="3"*
(18) *Title Color="Gelb" "Temperatur Art. Eingang"*
(19) *ValueCan="000060"*
(20) *Value Fmt="%3d" Unit="°C"*

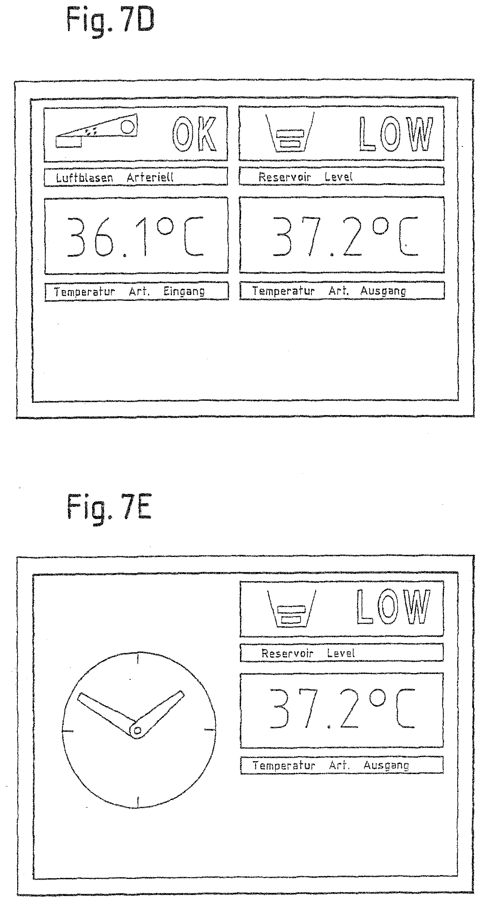

Entsprechend veranlassen die folgenden Konfigurationsdaten eine Anzeige in der rechten Hälfte der zweiten Zeile des Anzeige 8 der Anzeige/Bedieneinheit 6, wie Figur 7D zeigt, wobei die hier darzustellenden Werte von einem Temperatursensor mit der Identifikation 000059 stammen.
(21) *Displet="4"*
(22) *Title Color="Gelb" "Temperatur Art. Ausgang"*
(23) *ValueCan="000059"*
(24) *Value Fmt="%3d" Unit="°C"*

Die zuvor beschriebenen Konfigurationsdaten veranlassen die Anzeige/Bedieneinheit 6 ferner, den Bereich der Anzeige oberhalb der Titelzeilen als Eingabebereiche festzulegen. Das bedeutet, dass, wenn ein Benutzer die Oberfläche der Anzeige 8 der Anzeige/Bedieneinheit 6 im Bereich oberhalb einer der Titelzeilen berührt, eine Eingabe zu dem betreffenden Sensor entgegengenommen wird. Wenn beispielsweise aufgrund eines zu geringen Füllstands im Blutreservoir (vgl. Figur 7B) ein Alarm ausgelöst wurde, kann der Eingabebereich oberhalb der Titelzeile "Reservoir Level" ausgewertet werden, so dass eine Berührung dieses Bereichs durch den Benutzer als Bestätigung des Alarms angesehen wird.

In den Konfigurationsdaten können auch solche Daten vorgesehen werden, die Eingabebereiche ohne die Festlegung von Messwerteanzeigen bestimmen. Auf diese Weise können Schalter, Taster oder Regler dargestellt werden, die durch Berührung der berührungsempfindlichen Oberfläche der Anzeige/Bedieneinheit 6 betätigt werden.

Die verschiedenen Bereiche innerhalb der Anzeige einer Anzeige/Bedieneinheit 6 können auch verbunden werden, um größere zusammenhängende Anzeigenbereiche zu bilden. So zeigt zum Beispiel Fig. 7E eine konfigurierte Anzeige/Bedieneinheit, bei der die drei übereinander liegenden Bereiche in der linken Hälfte der Anzeige 8 zu einem Anzeigenbereich zusammengefasst wurden, um einen Anzeigenbereich zu schaffen, der die Darstellung einer analogen Uhr aufnehmen kann. Die Konfigurationsdaten, die die Konfigurationseinrichtung der Anzeige/Bedieneinheit 6 für diesen Anzeigeninhalt übermittelt, haben beispielhaft folgendes Format:
(25) *Displet="1,3,5"*
(26) *Displlcon Pic="ClockAnalog"*

Die von der Anzeigesteuereinrichtung 9 auf der Anzeige 8 der erfindungsgemäßen Anzeige/Bedieneinheit 6 darzustellenden Anzeigeninhalte sind vorzugsweise in einem Speicher der Anzeigesteuereinrichtung 9 abgelegt. Im Wesentlichen handelt es sich dabei um Ziffern und Buchstaben, sowie grafische Bildelemente, die Messwerte oder Betriebszustände, aber auch die Einheiten und Komponenten des medizintechnischen Geräts repräsentieren. Die Abspeicherung dieser Daten in der Anzeigesteuereinrichtung 9 führt dazu, dass von der Konfigurationseinrichtung 18 keine Daten für die Darstellung der Ziffern, Buchstaben und grafischen Bilddarstellungen übermittelt werden müssen. Diese Ausgestaltung einer erfindungsgemäßen Anzeige- und Bedienvorrichtung ist vorteilhaft, jedoch kann bei Steuerungssystemen auch eine Übertragung der Daten für die Anzeigeninhalte von der Konfigurationseinrichtung vorgesehen werden. In jedem Fall ist es vorteilhaft, wenn die Anzeigesteuereinrichtung 9 in der Lage ist, zusätzliche Daten für Anzeigeninhalte zu empfangen, in einem Speicher abzulegen und während des Betriebs für die Darstellung der Anzeige 8 der erfindungsgemäßen Anzeige/Bedieneinheit 6 zu verwenden. Dies erweitert die Einsetzbarkeit einer erfindungsgemäßen Anzeige/Bedieneinheit 6, da bei der Herstellung unbekannter Anzeigeninhalte nachträglich von der Konfigurationseinrichtung 18 an die Anzeige/Bedieneinheit 6 übertragen werden können. Vorzugsweise ist die Anzeigesteuereinrichtung 9 in diesem Fall in der Lage, die Kommunikationseinrichtung 18 über den Bus 15 zu vermitteln, welche Daten bereits in der Anzeigesteuereinrichtung 9 abgespeichert sind, so dass die Konfigurationseinrichtung 18 nur noch solche Daten für die Anzeigeninhalte überträgt, die nicht bereits in der Anzeigesteuereinheit 9 vorhanden sind.

Die hier beschriebene Vorgehensweise für den Aufbau, den Inhalt und das Ergebnis der Konfigurationsdaten sowie die Art des Bussystems dient als Beispiel und wirkt nicht beschränkend auf das Prinzip der erfindungsgemäßen Anzeige- und Bedienvorrichtung, die auch mit anderen Konfigurationsdaten und anderen Bussystemen realisierbar ist.

In dem beschriebenen Ausführungsbeispiel bestimmte die Position, an der eine Anzeige/Bedieneinheit 6 an die Basiseinheit 7 und damit an den Bus 15 angeschlossen wurde, welche Funktion der Anzeige/Bedieneinheit zugewiesen wurde. Diese Variante ist vorteilhaft, da auf dem Bedienpaneel Werte und Bedienelemente immer an derselben Stelle vorkommen. In einer alternativen, nicht beanspruchten Variante kann vorgesehen werden, dass Konfigurationsdaten der Reihe nach den Anzeige/Bedienelementen übermittelt werden, sodass der Benutzer festlegen kann, an welcher Stelle des Bedienpaneels welche Funktion bereitgestellt wird.

## Patentansprüche

1. Anzeige- und Steuervorrichtung (2) für ein medizintechnisches Gerät, umfassend:
eine Basiseinheit (7) und mehrere in ihrem Aufbau identische GUI-Einheiten (6, 6a, 6b, 6c, 6d, 6e, 6f) als Steckmodul, die an verschiedenen Positionen der Basiseinheit (7) anbringbar sind,
wobei die Basiseinheit (7) eine Konfigurationseinrichtung (18) aufweist, die
- über ein Bussystem in der Basiseinheit (7) mit der jeweiligen GUI-Einheit (6a, 6b, 6c, 6d, 6e, 6f) verbunden ist,
**dadurch gekennzeichnet, dass**
die identischen GUI-Einheiten (6, 6a, 6b, 6c, 6d, 6e, 6f) identische Touch-Screen-GUI-Einheiten (6, 6a, 6b, 6c, 6d, 6e, 6f) sind, und
die Konfigurationseinrichtung (18) ferner
- ausgebildet ist, Konfigurationsdaten für eine Anzeige- und Bedienfunktion der jeweiligen Touch-Screen-GUI-Einheit (6, 6a, 6b, 6c, 6d, 6e, 6f) festzulegen und bei Anschliessen einer Touch-Screen-GUI-Einheit an das Bussystem solche Konfigurationsdaten an die Touch-Screen-GUI-Einheit zu übermitteln, welche der Ansteckposition auf der Basiseinheit (7) entsprechen, wobei die jeweilige Touch-Screen-GUI-Einheit ausgebildet ist, über das Bussystem die Konfigurationsdaten von der Konfigurationseinrichtung (18) zu erhalten,
wobei erst die Konfigurationsdaten festlegen, für welche Aufgabe in der Gesamtheit der Anzeige- und Bedienvorrichtung die Touch-Screen-GUI-Einheit (6, 6a, 6b, 6c, 6d, 6e, 6f) eingesetzt wird indem die Konfigurationsdaten die Anzeige- und Bedienfunktion der jeweiligen Touch-Screen-GUI-Einheit (6, 6a, 6b, 6c, 6d, 6e, 6f) durch Anzeigeinhalte und Eingabebereiche bestimmen, wobei mit Hilfe der Anzeigefunktion der Betriebszustand des medizintechnischen Geräts erfassbar ist und mit Hilfe der Bedienfunktion in den Betrieb des medizintechnischen Geräts eingreifbar ist.

2. Anzeige- und Steuervorrichtung nach Anspruch 1, wobei
die Konfigurationseinrichtung (18) ausgebildet ist, unmittelbar nach einem Anschließen einer der Touch-Screen-GUI-Einheiten an das Bussystem, die Konfigurationsdaten an die Touch-Screen-GUI-Einheit zu übermitteln.

3. Anzeige- und Steuervorrichtung nach Anspruch 1 oder 2, wobei die Konfigurationseinrichtung (18) während des Betriebs der Anzeige- und Steuervorrichtung (2) keine weitere Funktion übernimmt.

4. Anzeige- und Steuervorrichtung nach einem der Ansprüche 1 - 2, wobei die Konfigurationseinrichtung (18) während des Betriebs der Anzeige- und Steuervorrichtung (2) keine weitere Funktion übernimmt, so dass ihr Ausfall während des Betriebs nach abgeschlossener Konfiguration der Anzeige- und Bedienfunktion zunächst keine Beeinträchtigung der Gesamtfunktion der Anzeige- und Steuervorrichtung (2) darstellt.

5. Anzeige- und Steuervorrichtung nach einem der Ansprüche 1 - 4, wobei
die Touch-Screen-GUI-Einheit (6, 6a, 6b, 6c, 6d, 6e, 6f) umfasst:
- eine Anzeigeeinrichtung (8), die eine Vielzahl von ansteuerbaren Bildpunkten aufweist,
- eine Anzeigesteuereinrichtung (9), die ausgebildet ist, die
Bildpunkte der Anzeigeeinrichtung auf der Grundlage von zugeführten Daten anzusteuern,
- eine transparente Eingabeeinrichtung (11), die auf der einem Betrachter zugewandten Oberfläche der Anzeigeeinrichtung (8) angeordnet ist,
- eine Eingabeauswerteeinrichtung (12), die ausgebildet ist, die über die Eingabeeinrichtung erfolgenden Eingaben auszuwerten, und
- eine Anschlusseinrichtung (13), die ausgebildet ist, mit der Anzeigesteuereinrichtung (9) und der Eingabeauswerteeinrichtung (12) in Verbindung zu stehen und mit der die Touch-Screen-GUI-Einheit (6, 6a, 6b, 6c, 6d, 6e, 6f) an einen elektrischen Bus (15) anschließbar ist, und
wobei die Basiseinheit (7) umfasst
- einen elektrischen Bus (15) für die Kommunikation von an den elektrischen Bus angeschlossenen Einheiten und Verbindungseinrichtungen (16), an die die Touch-Screen-GUI-Einheiten (6, 6a, 6b, 6c, 6d, 6e, 6f) mit Hilfe der Anschlusseinrichtung (13) an den elektrischen Bus (15) anschließbar sind, und
- die Konfigurationseinrichtung (18), die mit dem elektrischen Bus (15) verbunden ist und die ausgebildet ist, nach dem Anschließen einer Touch-Screen-GUI-Einheit (6, 6a, 6b, 6c, 6d, 6e, 6f) an den elektrischen Bus (15) der Touch-Screen-GUI-Einheit (6, 6a, 6b, 6c, 6d, 6e, 6f) über den elektrischen Bus (15) die Konfigurationsdaten zu übermitteln.

6. Anzeige- und Steuervorrichtung nach Anspruch 5, **dadurch gekennzeichnet, dass** für jede der Verbindungseinrichtungen (16) der Basiseinheit (7) in der Konfigurationseinrichtung (18) festgelegt ist, welche Konfigurationsdaten zu einer an der jeweiligen Verbindungseinrichtung (16) angeschlossenen Touch-Screen-GUI-Einheit (6, 6a, 6b, 6c, 6d, 6e, 6f) übertragen werden.

7. Anzeige- und Steuervorrichtung nach Anspruch 5 oder 6, **dadurch gekennzeichnet, dass** in der Anzeige (8) der Touch-Screen-GUI-Einheit (6, 6a, 6b, 6c, 6d, 6e, 6f) mehrere Bereiche für die Anzeige von Anzeigeninhalten und für die Entgegennahme von Eingaben logisch festgelegt sind.

8. Anzeige- und Steuervorrichtung nach einem der Ansprüche 5 - 7,
**dadurch gekennzeichnet, dass** die Touch-Screen-GUI-Einheiten (6, 6a, 6b, 6c, 6d, 6e, 6f) durch die Verbindung zwischen Anschlusseinrichtung (13) und Verbindungseinrichtung (16) an der Basiseinheit (7) gehaltert werden.

9. Anzeige- und Steuervorrichtung nach einem der Ansprüche 5 - 8, **dadurch gekennzeichnet, dass** die Touch-Screen-GUI-Einheiten (6, 6a, 6b, 6c, 6d, 6e, 6f) durch zusätzliche Halterungselemente an der Basiseinheit (7) gehaltert werden.

10. Anzeige- und Steuervorrichtung nach einem der Ansprüche 5 - 9, **dadurch gekennzeichnet, dass** Daten für die Anzeige von Ziffern, Buchstaben und grafischen Bilddarstellungen in der Anzeigensteuereinrichtung (9) der Touch-Screen-GUI-Einheit (6, 6a, 6b, 6c, 6d, 6e, 6f) abgespeichert sind.

11. Anzeige- und Steuervorrichtung nach einem der Ansprüche 5 - 10, **dadurch gekennzeichnet, dass** die Touch-Screen-GUI-Einheit (6, 6a, 6b, 6c, 6d, 6e, 6f) und die Konfigurationseinrichtung (18) dafür ausgelegt sind, dass Daten für die Anzeigeninhalte von der Konfigurationseinrichtung (18) an die Touch-Screen-GUI-Einheit (6, 6a, 6b, 6c, 6d, 6e, 6f) übertragbar und in der Touch-Screen-GUI-Einheit (6, 6a, 6b, 6c, 6d, 6e, 6f) speicherbar sind.

12. Anzeige- und Steuervorrichtung nach einem der Ansprüche 5 - 11, **dadurch gekennzeichnet, dass** die Touch-Screen-GUI-Einheit (6, 6a, 6b, 6c, 6d, 6e, 6f) ausgebildet ist, der Konfigurationseinrichtung (18) zu übermitteln, welche Daten für Anzeigeninhalte in der Anzeigensteuereinrichtung (9) gespeichert sind.

13. Anzeige- und Steuervorrichtung nach einem der Ansprüche 5 - 12, **dadurch gekennzeichnet, dass** eine Buskommunikationsvorrichtung (14) vorgesehen ist, über die die Anzeigensteuereinrichtung (9) und die Eingabeauswerteeinrichtung (12) an den elektrischen Bus (15) angeschlossen sind.

14. Anzeige- und Steuervorrichtung nach einem der Ansprüche 5 - 13, **dadurch gekennzeichnet, dass** die Touch-Screen-GUI-Einheit (6, 6a, 6b, 6c, 6d, 6e, 6f) eingerichtet ist, Informationen anzuzeigen und Eingaben entgegenzunehmen, und wobei für die Touch-Screen-GUI-Einheit neben einem Ein/Aus-Schalter keine weiteren Bedienelemente vorgesehen sind.

## Claims

1. Display and control device (2) for medical equipment comprising:
a base unit (7) and several display/operating units (6, 6a, 6b, 6c, 6d, 6e, 6f), identical in their structure as plug-in modules, which can be attached in different positions on the base unit (7),
wherein the base unit (7) has a configuration unit (18) which
- is connected via a bus system in the base unit (7) with the respective display/operating unit (6a, 6b, 6c, 6d, 6e, 6f),
**characterised in that**
the identical display/operating units (6, 6a, 6b, 6c, 6d, 6e, 6f) are identical touchscreen display/operating units (6, 6a, 6b, 6c, 6d, 6e, 6f), and
the configuration unit (18)
- is further configured to define configuration data for a display and operating function of the respective touchscreen display/operating unit (6, 6a, 6b, 6c, 6d, 6e, 6f) and, on connection of a touchscreen display/operating unit to the bus system, to communicate configuration data to the touchscreen display/operating unit which correspond to the plug-in position on the base unit (7), wherein the respective touchscreen display/operating unit is configured to receive the configuration data from the configuration unit (18) via the bus system,
wherein it is the configuration data which define for which function, within the totality of the display and operating device, the touchscreen display/operating unit (6, 6a, 6b, 6c, 6d, 6e, 6f) is used **in that** the configuration data define the display and operating function of the respective touchscreen display/operating unit (6, 6a, 6b, 6c, 6d, 6e, 6f) through display contents and input ranges, wherein the operating status of the medical equipment is identified with the aid of the display function and it is possible to intervene in the operation of the medical equipment with the aid of the operating function.

2. Display and control device according to claim 1, wherein
the configuration unit (18) is configured to communicate the configuration data to the touchscreen display/operating unit immediately following connection of one of the touchscreen display/operating units to the bus system.

3. Display and control device according to claim 1 or 2, wherein the configuration unit (18) does not perform any further function during operation of the display and control device (2).

4. Display and control device according to one of the claims 1 - 2, wherein the configuration unit (18) does not perform any further function during operation of the display and control device (2), so that following completed configuration of the display and operating function its failure during operation does not initially affect the overall function of the display and control device (2).

5. Display and control device according to one of the claims 1 - 4, wherein
the touchscreen display/operating unit (6, 6a, 6b, 6c, 6d, 6e, 6f) comprises:
- a display device (8) which comprises a plurality of controllable pixels,
- a display control device (9) which is configured to control the pixels of the display device on the basis of supplied data,
- - a transparent input device (11) which is arranged on the surface of the display device (8) facing towards an observer,
- an input evaluating device (12) which is configured to evaluate the inputs made via the inputs, and
- a terminal device (13) which is configured to be connected with the display control device (9) and the input evaluating device (12) and with which the touchscreen display/operating unit (6, 6a, 6b, 6c, 6d, 6e, 6f) can be connected to an electrical bus (15), and
wherein the base unit (7) comprises
- an electrical bus (15) for communication with units connected to the electrical bus and connecting devices (16) to which the touchscreen display/operating units (6, 6a, 6b, 6c, 6d, 6e, 6f) can be connected with the aid of the terminal device (13) on the electrical bus (15), and
- the configuration unit (18), which is connected to the electrical bus (15) and which is configured, immediately following connection of a touchscreen display/operating unit (6, 6a, 6b, 6c, 6d, 6e, 6f) to the electrical bus (15), to communicate the configuration data to the touchscreen display/operating unit (6, 6a, 6b, 6c, 6d, 6e, 6f) via the electrical bus (15).

6. Display and control device according to claim 5, **characterised in that**, for each of the connecting devices (16) of the base unit (7), it is defined in the configuration unit (18) which configuration data are transmitted to a touchscreen display/operating unit (6, 6a, 6b, 6c, 6d, 6e, 6f) connected to the respective connecting device (16).

7. Display and control device according to claim 5 or 6, **characterised in that** in the display (8) of the touchscreen display/operating unit (6, 6a, 6b, 6c, 6d, 6e, 6f) a plurality of areas are logically fixed for the display of display contents and for receiving inputs.

8. Display and control device according to one of the claims 5 - 7,
**characterised in that** the touchscreen display/operating units (6, 6a, 6b, 6c, 6d, 6e, 6f) can be supported on the base unit (7) by the connection between terminal device (13) and connecting device (16).

9. Display and control device according to one of the claims 5 - 8, **characterised in that** the touchscreen display/operating units (6, 6a, 6b, 6c, 6d, 6e, 6f) are supported on the base unit (7) by additional support elements.

10. Display and control device according to one of the claims 5 - 9, **characterised in that** data for the display of figures, letters and graphic image representations are stored in the display control device (9) of the touchscreen display/operating unit (6, 6a, 6b, 6c, 6d, 6e, 6f).

11. Display and control device according to one of the claims 5 - 10, **characterised in that** the touchscreen display/operating unit (6, 6a, 6b, 6c, 6d, 6e, 6f) and the configuration unit (18) are designed in order that data for the display contents can be transmitted from the configuration unit (18) to the touchscreen display/operating unit (6, 6a, 6b, 6c, 6d, 6e, 6f) and stored in the touchscreen display/operating unit (6, 6a, 6b, 6c, 6d, 6e, 6f).

12. Display and control device according to one of the claims 5 - 11, **characterised in that** the touchscreen display/operating unit (6, 6a, 6b, 6c, 6d, 6e, 6f) is configured to communicate to the configuration unit (18) which data for display contents are stored in the display control device (9).

13. Display and control device according to one of the claims 5 - 12, **characterised in that** a bus communication device (14) is provided via which the display control device (9) and the input evaluating device (12) are connected to the electrical bus (15).

14. Display and control device according to one of the claims 5 - 13, **characterised in that** the touchscreen display/operating unit (6, 6a, 6b, 6c, 6d, 6e, 6f) is configured to display information and receive inputs, and wherein, apart from an on/off switch, no further operating elements are provided for the touchscreen display/operating unit.

## Revendications

1. Dispositif d'affichage et de commande (2) pour un appareil médicotechnique comprenant :
une unité de base (7) et plusieurs unités GUI de structure identique (6, 6a, 6b, 6c, 6d, 6e, 6f) comme module d'enfichage, qui peuvent être disposées dans diverses positions de l'unité de base (7),
dans lequel l'unité de base (7) présente un dispositif de configuration (18), qui
- est raccordé à l'unité GUI respective (6a, 6b, 6c, 6d, 6e, 6f) via un système de bus dans l'unité de base (7),
**caractérisé en ce que**
les unités GUI identiques (6, 6a, 6b, 6c, 6d, 6e, 6f) sont des unités GUI pour écran tactile identiques (6, 6a, 6b, 6c, 6d, 6e, 6f), et
le dispositif de configuration (18) est en outre
- aménagé pour établir des données de configuration pour une fonction d'affichage et de service de l'unité GUI pour écran tactile respective (6, 6a, 6b, 6c, 6d, 6e, 6f) et transmettre, lors du raccordement d'une unité GUI pour écran tactile au système de bus, de telles données de configuration à l'unité GUI pour écran tactile, qui correspondent à la position amovible sur l'unité de base (7), dans lequel l'unité GUI pour écran tactile respective est aménagée pour maintenir les données de configuration du dispositif de configuration (18) via le système de bus,
dans lequel avant tout les données de configuration établissent à quelle fin l'unité GUI pour écran tactile (6, 6a, 6b, 6c, 6d, 6e, 6f) est utilisée dans la totalité du dispositif d'affichage et de commande en déterminant par les données de configuration la fonction d'affichage et de service de l'unité GUI pour écran tactile respective (6, 6a, 6b, 6c, 6d, 6e, 6f) par des contenus d'affichage et des zones d'entrée, dans lequel l'état de marche de l'appareil médicotechnique peut être saisi à l'aide de la fonction d'affichage et peut participer au fonctionnement de l'appareil médicotechnique à l'aide de la fonction de service.

2. Dispositif d'affichage et de commande selon la revendication 1, dans lequel :
le dispositif de configuration (18) est aménagé pour transmettre les données de configuration à l'unité GUI pour écran tactile directement après le raccordement de l'une des unités GUI pour écran tactile au système de bus.

3. Dispositif d'affichage et de commande selon la revendication 1 ou 2, dans lequel :
le dispositif de configuration (18) ne prend en charge aucune autre fonction pendant le fonctionnement du dispositif d'affichage et de commande (2).

4. Dispositif d'affichage et de commande selon l'une quelconque des revendications 1-2, dans lequel le dispositif de configuration (18) ne prend en charge aucune autre fonction pendant le fonctionnement du dispositif d'affichage et de commande (2) si bien que sa défaillance au cours du fonctionnement, une fois terminée la configuration, de la fonction d'affichage et de commande ne représente tout d'abord aucun préjudice à la fonction totale du dispositif d'affichage et de commande (2).

5. Dispositif d'affichage et de commande selon l'une quelconque des revendications 1-4, dans lequel
l'unité GUI pour écran tactile (6, 6a, 6b, 6c, 6d, 6e, 6f) comprend :
- un dispositif d'affichage (8) qui présente une pluralité de pixels pouvant être commandés,
- un dispositif de commande d'affichage (9) qui est aménagé pour commander les pixels du dispositif d'affichage sur la base des données saisies,
- un dispositif d'entrée transparent (11) qui est agencé sur la surface du dispositif d'affichage (8) tournée vers un observateur,
- un dispositif d'évaluation d'entrée (12) qui est aménagé pour évaluer les entrées qui se produisent via le dispositif d'entrée, et
- un dispositif de raccordement (13) qui est aménagé pour se lier au dispositif de commande d'affichage (9) et au dispositif d'évaluation d'entrée (12) et auquel l'unité GUI pour écran tactile (6, 6a, 6b, 6c, 6d, 6e, 6f) peut être raccordée à un bus électrique (15), et
dans lequel l'unité de base (7) comprend
- un bus électrique (15) pour la communication d'unités raccordées au bus électrique et de dispositifs de liaison (16), auxquels les unités GUI pour écran tactile (6, 6a, 6b, 6c, 6d, 6e, 6f) peuvent être raccordées au bus électrique (15) à l'aide du dispositif de raccordement (13) et
- le dispositif de configuration (18) qui est lié au bus électrique (15) et qui est aménagé pour transmettre les données de configuration, après le raccordement d'une unité GUI pour écran tactile (6, 6a, 6b, 6c, 6d, 6e, 6f) au bus électrique (15) de l'unité GUI pour écran tactile (6, 6a, 6b, 6c, 6d, 6e, 6f) via le bus électrique (15).

6. Dispositif d'affichage et de commande selon la revendication 5, **caractérisé en ce que**, pour chacun des dispositifs de liaison (16) de l'unité de base (7), on établit dans le dispositif de configuration (18) quelles données de configuration sont transmises à une unité GUI pour écran tactile (6, 6a, 6b, 6c, 6d, 6e, 6f) raccordée au dispositif de liaison respectif (16).

7. Dispositif d'affichage et de commande selon la revendication 5 ou 6, **caractérisé en ce que** l'on établit de manière logique dans l'affichage (8) de l'unité GUI pour écran tactile (6, 6a, 6b, 6c, 6d, 6e, 6f) plusieurs zones pour l'affichage de contenus d'affichage et pour la réception d'entrées.

8. Dispositif d'affichage et de commande selon l'une quelconque des revendications 5-7,
**caractérisé en ce que** les unités GUI pour écran tactile (6, 6a, 6b, 6c, 6d, 6e, 6f) sont fixées par la liaison entre le dispositif de raccordement (13) et le dispositif de liaison (16) à l'unité de base (7).

9. Dispositif d'affichage et de commande selon l'une quelconque des revendications 5-8, **caractérisé en ce que** les unités GUI pour écran tactile (6, 6a, 6b, 6c, 6d, 6e, 6f) sont fixées par des éléments de fixation supplémentaires à l'unité de base (7).

10. Dispositif d'affichage et de commande selon l'une quelconque des revendications 5-9, **caractérisé en ce que** des données pour l'affichage de chiffres, de lettres et de représentations d'images graphiques sont enregistrées dans le dispositif de commande d'affichage (9) de l'unité GUI pour écran tactile (6, 6a, 6b, 6c, 6d, 6e, 6f).

11. Dispositif d'affichage et de commande selon l'une quelconque des revendications 5-10, **caractérisé en ce que** l'unité GUI pour écran tactile (6, 6a, 6b, 6c, 6d, 6e, 6f) et le dispositif de configuration (18) sont conçus pour pouvoir transférer des données pour les contenus d'affichage du dispositif de configuration (18) à l'unité GUI pour écran tactile (6, 6a, 6b, 6c, 6d, 6e, 6f) et pouvoir les enregistrer dans l'unité GUI pour écran tactile (6, 6a, 6b, 6c, 6d, 6e, 6f).

12. Dispositif d'affichage et de commande selon l'une quelconque des revendications 5-11, **caractérisé en ce que** l'unité GUI pour écran tactile (6, 6a, 6b, 6c, 6d, 6e, 6f) est aménagée pour transmettre au dispositif de configuration (18) quelles données pour les contenus d'affichage sont enregistrées dans le dispositif de commande d'affichage (9).

13. Dispositif d'affichage et de commande selon l'une quelconque des revendications 5-12, **caractérisé en ce qu'**il est prévu un dispositif de communication par bus (14), par le biais duquel le dispositif de commande d'affichage (9) et le dispositif d'évaluation d'entrée (12) sont raccordés au bus électrique (15).

14. Dispositif d'affichage et de commande selon l'une quelconque des revendications 5-13, **caractérisé en ce que** l'unité GUI pour écran tactile (6, 6a, 6b, 6c, 6d, 6e, 6f) est configurée pour afficher des informations et recevoir des entrées et dans lequel aucun autre élément de service n'est prévu pour l'unité GUI pour écran tactile en dehors d'un commutateur marche/arrêt.
